# EUROPÄISCHE PATENTSCHRIFT

(11) **EP 0 518 454 B1**
(45) Veröffentlichungstag und Bekanntmachung des Hinweises auf die Patenterteilung: **09.08.1995**
(21) Anmeldenummer: 92250136.6
(22) Anmeldetag: 31.05.1992
(51) Int. Cl.: A61K 6/06

(54) **Verwendung einer Mischung zur Herstellung opaker Dentalwerkstoffe**
Use of a composition for the preparation of an opaque dental material
Utilisation d'une composition pour la fabrication d'un matériau dentaire opaque

(30) Priorität: 13.06.1991 DE 4119483
(43) Veröffentlichungstag der Anmeldung: 16.12.1992
(73) Patentinhaber: IVOCLAR AG, FL-9494 Schaan (LI)
(72) Erfinder: Rheinberger, Volker, Dr., FL-9490 Vaduz (LI); Wollwage, Peter, FL-9493 Mauren (LI)
(74) Vertreter: UEXKÜLL & STOLBERG

(56) Entgegenhaltungen:
- EP-A- 0 280 985
- DE-A- 1 491 042
- FR-A- 2 454 796
- GB-A- 897 686
- JOURNAL OF THE AMERICAN CERAMIC SOCIETY Bd. 69, Nr. 4, 1986, COLUMBUS, OHIO, USA, Seiten C-75 -C-77 E. MORENA E.A."TOUGHENING OF DENTAL PORCELAIN BY TETRAGONAL ZR02 ADDITIONS"

## Beschreibung

Die Erfindung betrifft die Verwendung einer Mischung zur Herstellung einer Grundiermasse oder als Grundiermasse.

Beim Herstellen von Zahnkronen und -brücken aus Metall, die mit Kunststoff oder Keramik verblendet werden, ist es notwendig, das Metallgerüst mit einer Grundiermasse - einem sogenannten Opaker - zu überziehen, damit der dunkle metallische Untergrund abgedeckt und ein Durchschimmern des Metalls vermieden wird. Ohne einen solchen Opaker kann die Farbe der fertigen Verblendung, die möglichst mit der Farbe des Restgebisses übereinstimmen soll, nicht exakt wiedergegeben werden.

Aus der DE-PS 33 32 179 sind Grundiermassen für dentale Zwecke auf der Basis von polymerisierbaren Methacrylsäureestern bekannt, die eine Mischung von Titandioxid und Zirkoniumdioxid als Pigment enthalten, wobei ein beträchtlicher Titandioxidgehalt, nämlich von bis zu 50 Gew.-%, vorgesehen ist. Die Grundiermassen der Beispiele enthalten 25 Gew.-% Titandioxid. Sie werden zum Abdecken von Metallgerüsten für Kronen mit Kunststoffverblendungen eingesetzt.

Aus dem in dieser Druckschrift in Spalte 2 zitierten Stand der-Technik, nämlich Kirk-Othmer, Encyclopedia of Chemical Technology, 2. Auflage, New York (1970), Band 22, 651-653 geht hervor, daß die Verwendung von Zirkondioxid als keramisches Trübungsmittel und als Pigment oder Trübungsmittel für keramische Glasuren und Emails bekannt war. Es findet sich aber keine Anregung, Zirkondioxid als Zusatz für einen metallkeramischen Opaker, d.h. für eine keramische, insbesondere glaskeramische Grundiermasse zum Überziehen von Metallkronen oder -brücken einzusetzen, wobei zu bedenken ist, daß an einen derartigen Opaker spezielle Anforderungen gestellt werden. Zum Beispiel muß ein solcher Opaker in dünnen Schichten von höchstens 0,5 mm auf eine Krone aufgebracht und anschließend eingebrannt werden können, wobei nach dem Brand die Krone vollständig durch die hellschimmernde Opakerschicht abgedeckt sein soll.

Die DE-PS 37 43 609 beschreibt eine lichtdurchlässige Glaskeramik, welche feine Kristalle von Calciumphosphat umfaßt, die gleichförmig in einer Glasmatrix auf Basis von SiO₂-AlO₂-ZrO₂ dispergiert sind. Sie hat Bedeutung als Biomaterial und vorteilhafte thermische, elektrische und mechanische Eigenschaften. Die Glaskeramik ist transparent oder halbtransparent. Zirkondioxid wird als Kern- oder Keimbildungsmittel zugesetzt. Die Glaskeramik kann als Haft- oder Bindemittel für Keramikmassenmaterialien oder Metalle verwendet werden. In dieser Veröffentlichung findet sich kein Hinweis auf die Verwendung einer zirkondioxidhaltigen Glaskeramik als Opaker für den dentalen Einsatzbereich. Wesentlich ist ganz im Gegenteil die Lichtdurchlässigkeit der Glaskeramik, weshalb diese Druckschrift genau das Gegenteil von dem aussagt, was der Fachmann von einem Opaker erwartet.

Die EP-PS 119 062 offenbart pastenartige Materialien aus Porzellanpulver und Wasser mit einem Zusatz von Keramikteilchen einer Teilchengröße unter 0,3 »m. Die Materialien können als Opaker für Kronen mit Porzellanverblendungen eingesetzt werden.

Aus der DE-OS 3 902 771 ist eine aufbrennbare silikatische Masse zur Herstellung von Zahnersatz bekannt, die als Verbundmaterial und zur Maskierung der metallfarbenen Legierungsoberfläche dient. Zu lösende Aufgabe sollte sein, eine aus einer leicht fließenden Grundschmelze und einem Opakeranteil bestehende silikatische Masse anzugeben, deren Zusammensetzung und Ausdehnungskoeffizient derart eingestellt sind, daß sie auf ein Dentalprothesenteil, das aus Edelmetallegierungen des Silber-Palladiumtyps oder aus Dental-Nichtmetallegierungen der Grundtypen Nickel-Chrom und Kobalt-Chrom hergestellt sein kann, aufbrennbar ist und nach deren Silanisierung einen spaltfreien Verbund zu einem Dentalkunststoff bildet. Die hier vorgeschlagene silikatische Masse zeichnet sich durch einen hohen Titandioxidanteil (15,0 - 16,5 Gew.-%) und durch verhältnismäßig geringe Zirkondioxidanteile (4 bis 5 Gew.-%) aus.

Die Nachteile der Opaker der DE-PS 33 32 179, EP-PS 119 062 und DE-OS 39 02 771 sind mangelnde Deckfähigkeit und Haftung. Insbesondere im Bereich der Kronenränder kommt es nach dem Brand der bekannten Opaker zu unerwünschten Verfärbungen, welche die Kosmetik der nachfolgenden Verblendungen stark beeinträchtigen. Dies gilt auch für die seit längerer Zeit im Handel befindlichen Opaker, die dem Fachmann bekannt sind.

Es ist die Aufgabe der Erfindung, die Verwendung einer pulverförmigen Mischung zur Herstellung einer Grundiermasse für dentale Zwecke,nämlich zum Überziehen oder Beschichten von dentalen Metallgerüsten vor der Verblendung zu ermöglichen und darüber hinaus eine Grundiermasse insbesondere für Keramikverblendungen mit verbesserter Deckfähigkeit und Haftung bereitzustellen, deren Haftung sowohl am Metall als auch an der Verblendkeramik ausgezeichnet ist, die leicht appliziert und eingebrannt werden kann und die insbesondere im Bereich der Kronenränder das Metall zuverlässig abdeckt. Ferner sollen die vor allem bei NE-Legierungen (Nicht-Edelmetall-Legierungen) auftretenden Verfärbungen nach dem Brand vermieden werden.

Es wurde nun überraschenderweise gefunden, daß man durch Zusatz von größeren Mengen Zirkondioxid als Trübungsmittel zu Glaspulvern Mischungen erhält, durch deren Verwendung sich Opaker mit hervorragenden Eigenschaften herstellen lassen, die eine gute Abdeckung des Metallgerüsts und gute Haftung am Metall und an der Verblendung gewährleisten.

Zur Lösung der erfindungsgemäßen Aufgaben wird die Verwendung von Mischungen gemäß den Ansprüchen 1 bis 12 vorgeschlagen.

Wenngleich aus dem genannten Stand der Technik hervorgeht, daß Zirkondioxid als Trübungsmittel für keramische Glasuren bekannt war, so ist doch die Verwendung von größeren Mengen Zirkondioxid als im wesentlichen alleiniger Trübungsmittel-Zusatz zu Glaspulvern zur Herstellung eines Opakers weder bekannt noch nahegelegt. Es war vielmehr überraschend, daß man bei Zusammensetzungen, die frei von größeren Anteilen von Titandioxid und/oder anderen Trübungsmitteln sind und als Trübungsmittel-Zusatz zu Glaspulvern Zirkondioxid enthalten, Opaker erhält, die sich durch hervorragende Eigenschaften, insbesondere hervorragende Deckfähigkeit und Haftung auszeichnen.

Die für die erfindungsgemäße Verwendung geeignete Mischung besteht im wesentlichen aus einem Matrix bildenden Glaspulver, das mit 10 bis 60 Gew.-% Zirkondioxid als Trübungsmittel vermischt wird. Die Zusammensetzung ist frei von größeren Anteilen an Titandioxid und/oder anderen Trübungsmitteln, d.h. in der Mischung aus Glaspulver und Zirkondioxid sollen höchstens 0 bis 5 Gew.-% Titandioxid und/oder andere Trübungsmittel enthalten sein.

Das Glas besteht vorzugsweise im wesentlichen aus den Oxiden von Natrium, Kalium, Calcium, Barium, Bor, Aluminium und/oder Silicium mit geringem Anteil von Titan, wobei das gegebenenfalls anwesende TiO₂ im Glas eingeschmolzen ist und nicht als Trübungsmittel, sondern als Keimbildner für die Kristallisation von Leucit fungiert. Ferner können noch Oxide von Ce und gegebenenfalls von P und Zr in dem Glas enthalten sein. Das Glas enthält vorzugsweise als Hauptbestandteile SiO₂, Al₂O₃ und K₂O, bevorzugt in Mengen von jeweils 40 bis 70 Gew.-%, 10 bis 25 Gew.-% und 8 bis 28 Gew.-%. Es können auch andere Gläser eingesetzt werden.

Zur Herstellung des Glases wird das Oxidgemisch geschmolzen, in Wasser abgeschreckt und in an sich bekannter Weise zu einem feinen Glaspulver aufbereitet.

Im einzelnen wird beispielsweise das geschmolzene Oxidgemisch in Wasser abgeschreckt und in einer Kugelmühle aufgemahlen. Danach wird das Glaspulver bei etwa 900°C getempert, wobei es zu einem Block zusammensintert, der in noch glühendem Zustand in Wasser abgeschreckt wird. Dabei bildet sich ein Granulat, das in der Kugelmühle in einer Alkohol-Wassermischung aufgemahlen wird. Hierbei entsteht ein Schlicker, der getrocknet und dann durch ein Sieb gegeben wird.

Dieses Glaspulver wird mit Zirkondioxid vermischt. Die Mischung wird im Verhältnis von maximal 9 Teilen Glas je Teil Zirkondioxid hergestellt. Bevorzugte Mischungsverhältnisse liegen zwischen 4:1 und 1:1,5.

Zur Herstellung des anwendungsfertigen Opakers vermengt man das Pulvergemisch mit einer Flüssigkeit. Beispielsweise kann das Pulver mit destilliertem Wasser auf einer Keramikplatte zu einem Opaker angemischt werden. Es können jedoch auch andere Flüssigkeiten wie Glycerin oder Glykol zum Anmischen verwendet werden.

In einer besonders bevorzugten Ausführungsform werden 3 Gewichtsteile Glas mit 2 Gewichtsteilen Zirkondioxid vermischt und mit weiteren 2 Gewichtsteilen Glycerin auf einem Dreiwalzenstuhl homogenisiert.

Zur Anwendung als Grundiermasse wird die entstehende Paste mit einem Pinsel auf eine Krone aufgetragen und bei Temperaturen zwischen 800 und 1000°C eingebrannt. Es entsteht eine deckende weiße Schicht, die auch an den Metallrändern keine Verfärbungen aufweist, sich durch eine hohe Deckkraft auszeichnet und eine gute Haftung zwischen Metall und Keramik bietet. Die Krone wird dann nach herkömmlichen Methoden mit Keramik verblendet.

Gegebenenfalls kann man der Pulvermischung Farbpigmente beimischen, um dem gebrannten Opaker eine zahnfarbenähnliche Farbe zu verleihen. Die Farbkörper können jedoch auch während des Homogenisierens zugegeben werden.

Ferner ist es möglich, dem Glaspulver andere bekannte Keramikpulver beizumischen, um den Wärmeausdehnungskoeffizienten (WAK) zu variieren. Bekanntlich ist der WAK-Wert ein wichtiger Parameter bei metallkeramischen Verblendarbeiten und muß zur Legierung, die verblendet werden soll, passen.

Die Zirkondioxidteilchen haben eine mittlere Teilchengröße von 1 »m ± 0,2. Außerdem haben die Zirkondioxidteilchen eine spezifische Oberfläche von 1 bis 20 m²/g und eine Reinheit von 99% ± 1.

Die folgenden Beispiele erläutern die Erfindung.

### Beispiel 1

Herstellung einer Schmelze A mit hohem WAK:
Es wurde auf übliche Weise ein Glas mit folgender Zusammensetzung erschmolzen:
5,95 Gew.-% Na₂O
13,86 Gew.-% K₂O
3,48 Gew.-% CaO
1,64 Gew.-% BaO
1,36 Gew.-% B₂O₃
16,09 Gew.-% Al₂O₃
0,37 Gew.-% TiO₂
57,25 Gew.-% SiO₂.

Dieses Glas wurde in Wasser abgeschreckt und in einer Kugelmühle aufgemahlen, bis die mittlere Körnung etwa 80 bis 100 »m betrug.

Danach wurde das Glaspulver bei 900°C eine Stunde getempert, wobei es zu einem Block zusammensinterte, der noch in glühendem Zustand in Wasser abgeschreckt wurde. Das dabei entstehende Granulat wurde in der Kugelmühle in einer Alkohol-Wasser-Mischung (1:1) bis zu einer mittleren Körnung von unter 20 »m aufgemahlen. Die Mahldauer richtet sich nach Größe und Art der Kugelmühle.

Beispielsweise wurden
200 g grobes Glaspulver
800 g Porzellankugeln mit 9 mm Durchmesser
100 g destilliertes Wasser
100 g Alkohol
in einer 1 l Prozellantrommel 8 Stunden auf Rollen laufengelassen. Der resultiertende Schlicker wurde bei 100°C getrocknet und zur Vermeidung von verklebten Brocken durch ein 60 »m Sieb gegeben.

60 g dieser Schmelze wurden mit 40 g Zirkondioxid mit einer mittleren Körnung von 1 »m, das unter der Bezeichnung "Zirkonoxid SC15" von der Firma Magnesium Elektron vertrieben wird, gemischt.

Diese Mischung wurde mit 40 g Glycerin zu einer Paste vermengt und auf einer Salbendreiwalze homogenisiert. Die homogenisierte Paste wurde auf eine Nickel-Chrom-Legierung mit einem Pinsel aufgestrichen und danach bei 960°C mit 2 Minuten Haltezeit gebrannt. Es entstand eine deckende weiße Mattschicht von sehr guter Haftfestigkeit, die anschließend mit keramischen Dentinmassen überschichtet werden konnte. Die Beschichtung zeigte auch an den Metallrändern keine Verfärbung.

Es wurden Proben gebrannt, um die Festigkeit und den WAK zu prüfen. Folgende Werte konnten gemessen werden:

| | |
|---|---|
| Biegefestigkeit | 143 ± 11 N/mm² |
| Biegemodul | 73000 ± 6000 N/mm² |
| WAK 100 - 500°C | 14,4 x 10⁻⁶/K |

### Beispiel 2

Unter Verwendung der in Beispiel 1 genannten Schmelze A wurde nach folgendem Rezept eine Paste hergestellt und wie in Beispiel 1 homogenisiert:
60 g Schmelze A
20 g Zirkondioxid SC15
15 g keramische Farbe 23264 ^{x}
5 g keramische Farbe 26077 ^{xx}
0,5 g keramische Farbe 14-R-481 ^{xxx}
2 g SiO₂, BET Oberfläche 200 ± 25 (Aerosil 200 ® Degussa)
40 g Glycerin.
^{x} orange-gelb auf der Basis von Zr und V-Oxid (Firma Degussa)
^{xx} gelb-braun auf der Basis von Zr, Cr, Fe, Al-Oxid (Firma Degussa)
^{xxx} dunkelbraun, auf der Basis von Si, Co, Ni, Zn, Fe, Cr, Mn-Oxid (Firma Ferro)

Es wurde eine cremige Paste erhalten, die sich mit dem Pinsel gut verstreichen ließ. Nach dem Brennen auf einer Nickel-Chrom-Legierung bei 960°C wurde eine deckende, zahnfarbene Schicht erhalten, deren Farbe überall gleich ist.

| | |
|---|---|
| Biegefestigkeit | 161 ± 23 N/mm² |
| Biegemodul | 67000 ± 2000 N/mm |
| WAK 100 - 500°C | 15 x 10⁻⁶/K. |

### Beispiel 3

Ein für Metallkeramik übliches Keramikpulver für Schmelzgestaltung der Firma Ivoclar mit einem WAK 100-600°C von 13,5 x 10⁻⁶/K, einem Glaspunkt von 585°C und einem Dilatometer-Erweichungs-Punkt von 600°C wurde naß auf eine mittlere Korngröße von < 20 »m aufgemahlen, getrocknet und durch Sieben wie in Beispiel 1 beschrieben deagglomeriert. Dieses Pulver wurde mit GM-DS bezeichnet.

Folgende Mischung wurde hergestellt:
35 g Schmelze A aus Beispiel 1
25 g GM-DS
21 g Zirkondioxid SC15
14 g keramische Farbe 23264
5 g keramische Farbe 26077

Die Mischung wurde in einer Kugelmühle innig gemischt. Die Verarbeitung erfolgte, indem 1 g dieser Mischung mit Wasser angemischt und mit einem Pinsel auf eine Nickel-Chrom-Legierung aufgetragen wurde. Es zeigte sich das gleiche Erscheinungsbild wie in Beispiel 2, nur daß eine glänzendere Oberfläche erhalten wurde. Die Prüfung des WAK-Wertes zwischen 100 und 500°C ergab 14,5 x 10⁻⁶/K, was zeigt, daß der WAK-Wert durch Zumischen von anderen keramischen Massen variiert werden kann.

### Beispiel 4 (Vergleichsbeispiel mit Zinndioxid)

Um die Deckfähigkeit mit anderen Trübungsmitteln zu vergleichen, wurde folgende Mischung hergestellt und wie in Beispiel 1 beschrieben aufgearbeitet:
60 g Schmelze A aus Beispiel 1
25 g geglühtes Zinndioxid
17 g keramische Farbe 23264
11 g keramische Farbe 26077
45 g Glycerin

Die Verarbeitung dieser Mischung ergab eine ungenügend deckende Schicht, bei der die schwarzen Oxide der Nickel-Chrom-Legierung die Farbe verfälschten. Die Brenntemperatur dieser Mischung lag durch den hohen Gehalt an Zinndioxid bereits bei 1100°C, was für die in der Dentaltechnik üblichen Aufbrennlegierungen an der oberen Grenze liegt. Die Mischung verfärbte sich außerdem beim Lagern am Licht.

### Beispiel 5 (Vergleich mit verschiedenen Trübungsmitteln)

Um die Deckfähigkeit mit Titandioxid zu vergleichen, mußte ein spezielles Glas erschmolzen und wie in Beispiel 1 für die Schmelze A angegeben aufgearbeitet werden. Die Schmelze mit der Bezeichnung Schmelze B hatte folgende Zusammensetzung:
7,12 g Na₂O
11,82 g K₂O
3,2 g CaO
1,51 g BaO
1,25 g B₂O₃
14,5 g Al₂O₃
0,34 g TiO₂
59,35 g SiO₂

Folgende Mischung wurde wie in Beispiel 1 hergestellt:
70 g Schmelze B
30 g Trübungsmittel
40 g Glycerin

Die folgende Tabelle enthält die eingesetzten Trübungsmittel und die Versuchsergebnisse bei der Verarbeitung auf Nickel-Chrom-Legierungen:

| Trübungsmittel | Verarbeitungskonsistenz | Deckfähigkeit | Verfärbung beim Brennen |
|---|---|---|---|
| Titandioxid AD | gut | sehr gut | stark grau |
| Titandioxid R-KB-2 | gut | sehr gut | grau-braun |
| Titandioxid RN-56 | gut | sehr gut | grau-braun |
| gefälltes Zinnoxid | pastös | ungenügend | weiß |
| Zirkondioxid > 1 »m | flüssig | ungenügend | weiß |
| Zirkonsilikat | pastös | ungenügend | weiß |

Die Verfärbungen bei den Proben mit Titandioxid waren ungleichmäßig und gegen den Metallrand deutlich stärker, so daß diese Verfärbung nach dem Brennen nicht akzeptiert werden konnte. Die Proben mit Zinnoxid, Zirkondioxid > 1 »m und Zirkonsilikat hatten eine ungenügende Deckfähigkeit und waren ebenfalls nicht akzeptabel.

## Patentansprüche

1. Verwendung einer Mischung zur Herstellung einer Grundiermasse zum Überziehen oder Beschichten einer metallischen Zahnkrone oder -brücke durch Anfeuchten mit einer Flüssigkeit, wobei die Mischung ein Matrix bildendes Glasspulver und als Trübungsmittel 10 bis 60 Gew.-% ZrO₂ enthält, die mittlere Teilchengröße von ZrO₂ 1 »m ± 0,2 ist und die Zirkondioxidteilchen eine spezifische Oberfläche von 1 bis 20 m²/g aufweisen.

2. Verwendung nach Anspruch 1, **dadurch gekennzeichnet,**
daß das Glaspulver im wesentlichen aus den Oxiden von Na, K, Ca, Ba, B, Al und Si besteht.

3. Verwendung nach Anspruch 1 oder 2, **dadurch gekennzeichnet,** daß das Glaspulver als Hauptbestandteile
- 40 bis 70 Gew.-% SiO₂,
- 10 bis 25 Gew.-% Al₂O₃ und
- 8 bis 20 Gew.-% K₂O
enthält.

4. Verwendung nach einem oder mehreren der vorhergehenden Ansprüche, **dadurch gekennzeichnet,** daß das Glaspulver außerdem Oxide von Ce sowie gegebenenfalls von P und Zr enthält.

5. Verwendung nach einem oder mehreren der vorhergehenden Ansprüche, **dadurch gekennzeichnet**, daß die Mischung außerdem höchstens 5 Gew.-% TiO₂ und/oder andere Trübungsmittel enthält.

6. Verwendung nach einem oder mehreren der vorhergehenden Ansprüche, **dadurch gekennzeichnet,** daß die Mischung 20 bis 45 Gew.-% ZrO₂ enthält.

7. Verwendung nach einem oder mehreren der vorhergehenden Ansprüche, **dadurch gekennzeichnet,** daß die Zirkondioxidteilchen eine Reinheit von 99% ± 1 haben.

8. Verwendung nach einem oder mehreren der vorhergehenden Ansprüche, **dadurch gekennzeichnet,** daß die Mischung außerdem Farbpigmente und/oder übliche Zusatzstoffe enthält.

9. Verwendung nach einem oder mehreren der vorhergehenden Ansprüche, **dadurch gekennzeichnet**, daß das Gewichtsverhältnis von Glaspulver zu Zirkondioxidtrübungsmittel in dem Bereich von 9:1 bis 1:1,5 liegt.

10. Verwendung nach Anspruch 1, **dadurch gekennzeichnet**, daß die Flüssigkeit destilliertes Wasser oder Glycerin ist.

11. Verwendung nach Anspruch 10, **dadurch gekennzeichnet**, daß die Mischung 3 Gewichtsteile Glas mit 2 Gewichtsteilen Zirkondioxid und 2 Gewichtsteilen Glycerin homogenisiert enthält.

12. Verwendung der Grundiermasse nach den Ansprüchen 1 bis 11 als Grundiermasse zum Überziehen oder Beschichten einer metallischen Zahnkrone oder -brücke.

## Claims

1. Use of a mixture for the preparation of a priming composition for covering or coating a metallic dental crown or bridge by moistening with a liquid, wherein the mixture comprises a matrix-forming glass powder and, as an opacifying agent, 10 to 60% by weight of ZrO₂, the average particle size of the ZrO₂ is 1 »m ± 0.2 and the zirconium dioxide particles have a specific surface area of 1 to 20 m²/g.

2. Use according to Claim 1, characterized in that the glass powder essentially comprises the oxides of Na, K, Ca, Ba, B, Al and Si.

3. Use according to Claim 1 or 2, characterized in that the glass powder comprises, as the main constituents,
- 40 to 70% by weight of SiO₂,
- 10 to 25% by weight of Al₂O₃ and
- 8 to 20% by weight of K₂O.

4. Use according to one or more of the preceding claims, characterized in that the glass powder furthermore comprises oxides of Ce, and if appropriate of P and Zr.

5. Use according to one or more of the preceding claims, characterized in that the mixture furthermore comprises not more than 5% by weight of TiO₂ and/or other opacifying agents.

6. Use according to one or more of the preceding claims, characterized in that the mixture comprises 20 to 45% by weight of ZrO₂.

7. Use according to one or more of the preceding claims, characterized in that the zirconium dioxide particles have a purity of 99% ± 1.

8. Use according to one or more of the preceding claims, characterized in that the mixture furthermore comprises coloured pigments and/or customary additives.

9. Use according to one or more of the preceding claims, characterized in that the weight ratio of glass powder to zirconium dioxide opacifying agent is in the range from 9:1 to 1:1.5.

10. Use according to Claim 1, characterized in that the liquid is distilled water or glycerol.

11. Use according to Claim 10, characterized in that the mixture comprises 3 parts by weight of glass with 2 parts by weight of zirconium dioxide and 2 parts by weight of glycerol in homogenized form.

12. Use of the priming composition according to Claims 1 to 11 as a priming composition for covering or coating a metallic dental crown or bridge.

## Revendications

1. Utilisation d'une composition pour la préparation d'une matière de placage pour le recouvrement ou le revêtement d'une couronne ou d'un bridge dentaire métallique par imprégnation avec un liquide, la composition contenant une poudre de verre constituant la matière de base et, en tant qu'opacifiant, de 10 à 60 % en poids de ZrO₂, la taille moyenne de particules du ZrO₂ étant de 1 »m ± 0,2 »m et les particules de dioxyde de zirconium présentant une surface spécifique de 1 à 20 m²/g.

2. Utilisation selon la revendication 1, caractérisée en ce que la poudre de verre consiste essentiellement en les oxydes de Na, K, Ca, Ba, B, Al et Si.

3. Utilisation selon la revendication 1 ou 2, caractérisée en ce que la poudre de verre contient, en tant que composants essentiels:
- 40 à 70 % en poids de SiO₂,
- 10 à 25 % en poids de Al₂O₃ et
- 8 à 20 % en poids de K₂O.

4. Utilisation selon une ou plusieurs des revendications précédentes, caractérisée en ce que la poudre de verre contient en outre des oxydes de Ce ainsi qu'éventuellement de P et Zr.

5. Utilisation selon une ou plusieurs des revendications précédentes, caractérisée en ce que la composition contient en outre au maximum 5 % en poids de TiO₂ et/ou d'autres opacifiants.

6. Utilisation selon une ou plusieurs des revendications précédentes, caractérisée en ce que la composition contient de 20 à 45 % en poids de ZrO₂.

7. Utilisation selon une ou plusieurs des revendications précédentes, caractérisée en ce que les particules de dioxyde de zirconium ont un degré de pureté de 99 % ± 1 %.

8. Utilisation selon une ou plusieurs des revendications précédentes, caractérisée en ce que la composition contient en outre des pigments colorés et/ou des additifs usuels.

9. Utilisation selon une ou plusieurs des revendications précédentes, caractérisée en ce que le rapport pondéral de la poudre de verre à l'opacifiant dioxyde de zirconium se situe dans la plage allant de 9:1 à 1:1,5.

10. Utilisation selon la revendication 1, caractérisée en ce que le liquide est l'eau distillée ou le glycérol.

11. Utilisation selon la revendication 10, caractérisée en ce que la composition contient homogénéisées 3 parties en poids de verre avec 2 parties en poids de dioxyde de zirconium et 2 parties en poids de glycérol.

12. Utilisation de la matière de placage selon les revendications 1 à 11, en tant que matière de placage pour le recouvrement ou le revêtement d'une couronne ou d'un bridge dentaire métallique.
